# EUROPÄISCHE PATENTSCHRIFT

(11) **EP 4 162 890 B1**
(45) Veröffentlichungstag und Bekanntmachung des Hinweises auf die Patenterteilung: **16.07.2025**
(21) Anmeldenummer: 21201714.9
(22) Anmeldetag: 08.10.2021
(51) Int. Cl.: A61B 18/04

(54) **INSTRUMENTENANSCHLUSSEINRICHTUNG UND VERFAHREN ZUR HERSTELLUNG DERSELBEN**
INSTRUMENT CONNECTION DEVICE AND METHOD FOR PRODUCING THE SAME
DISPOSITIF CONNECTEUR D'INSTRUMENT ET SON PROCÉDÉ DE FABRICATION

(43) Veröffentlichungstag der Anmeldung: 12.04.2023
(73) Patentinhaber: Erbe Elektromedizin GmbH, 72072 Tübingen (DE)
(72) Erfinder: HEYM, Johannes, 72070 Tübingen (DE); AHLBURG, Elmar, 72818 Trochtelfingen (DE)
(74) Vertreter: Rüger Abel Patentanwälte PartGmbB

(56) Entgegenhaltungen:
- WO-A1-2021/173453
- US-A- 5 409 008
- US-A1- 2015 374 435
- US-A1- 2018 042 462

## Beschreibung

Die Erfindung betrifft eine Instrumentenanschlusseinrichtung, insbesondere für Instrumente, die einer Gaszufuhr und einer Stromzufuhr bedürfen. Im Weiteren betrifft die Erfindung ein Verfahren zur Herstellung einer solchen Instrumentenanschlusseinrichtung.

Chirurgische Instrumente zur Behandlung menschlicher oder tierischer Körper, die zum Betrieb sowohl eine Gasversorgung als auch eine Stromversorgung benötigen, sind bekannt. Beispielsweise offenbart dazu die US 7,717,911 B2 ein als flexible Sonde ausgebildetes Instrument, das im Wesentlichen aus einem langen Schlauch besteht, durch dessen Lumen sich ein Draht erstreckt. An dem distalen Ende des Schlauches ist ein Ende des Drahts etwa zentrisch in dem Schlauch gehalten und bildet so eine Elektrode. In Betrieb wird eine hochfrequente Wechselspannung an den Draht gelegt und das Lumen des Schlauches von Argon durchströmt. Es bildet sich am distalen Ende der Sonde ein Plasmastrahl.

Zum Anschluss solcher Instrumente an ein entsprechendes Versorgungsgerät beschreibt die EP 1 515 659 B1 einen Stecker mit einem Gehäuse, in dem sich das proximale Ende der Sonde befindet. Der Schlauch ist dort an eine Gasweiche angeschlossen, die ein gasführendes Lumen aufweist, das den Schlauch mit einem Filter verbindet. Außerdem führt die Gasweiche eine elektrische Leitung aus dem Lumen des Schlauches heraus und dichtet dabei das Lumen des Schlauches nach außen ab.

Aus der US 5,409,008 ist ein Instrument mit länglichem Rohrschaft bekannt, der zur Durchleitung von Fluiden dient. In einem proximalen Abschnitt des Instruments ist ein Handstück vorgesehen, das radial aus dem Rohrschaft abgehende Kanäle aufweist, durch welche elektrische Leiter in den Rohrschaft führen. Weiter offenbart die US 2018/0042462 A1 ein Instrument mit einem länglichen Schaft, an dessen proximalen Ende ein kurzes Verbindungsstück aus Kunststoff vorgesehen ist. Ein elektrischer Draht ist durch die Wandung dieses Kunststoffteils geführt.

Diese Instrumentenanschlusseinrichtung hat sich grundsätzlich bewährt, bedingt aber einen erheblichen Fertigungsaufwand.

Es ist Aufgabe der Erfindung, eine einfach herzustellende und dennoch dauerhaft verlässliche Instrumentenanschlusseinrichtung zu schaffen.

Diese Aufgabe wird mit der Instrumentenanschlusseinrichtung nach Anspruch 1 gelöst. Außerdem trägt das Verfahren nach Anspruch 13 zur Lösung der Aufgabe insoweit bei, als es einen einfachen Weg zur Herstellung einer verlässlichen Instrumentenanschlusseinrichtung weist.

Die erfindungsgemäße Instrumentenanschlusseinrichtung umfasst eine Gasweiche, zu der ein hohlzylindrischer Körper mit einer flexiblen Wandung gehört. Die Wandung begrenzt einen Durchgangskanal, der konzentrisch zu einer Längs- oder Axialrichtung festgelegt ist. Diese Längsachse ist die Mittelachse des hohlzylindrischen Körpers. In der Wandung des hohlzylindrischen Körpers ist ein Stichloch vorgesehen, durch das ein Draht führt. Dieser Draht erstreckt sich im Weiteren in einen Schlauch, der ein Anschlussschlauch für das Instrument oder alternativ auch ein Teil des Instruments sein kann. Der Draht kann sich dabei durch das Lumen des Schlauchs erstrecken oder ganz oder teilweise, z.B. abschnittsweise, in dem Schlauchmaterial eingebettet sein. Außerdem kann der Draht axial (längs) und, falls gewünscht, auch quer beweglich in dem Lumen des Schlauches angeordnet oder alternativ axial fixiert in dem Schlauch angeordnet sein.

Der Schlauch ist in dem Durchgangskanal des flexiblen hohlzylindrischen Körpers an dessen Wandung anliegend angeordnet, so dass eine gasdichte Verbindung zwischen dem hohlzylindrischen Körper und dem Schlauch gebildet ist. Erfindungsgemäß liegt die Wandung des Körpers unter Vorspannung an dem Schlauch an. Insbesondere werden Verbindungen als gasdicht angesehen, wenn bei einem Druckunterschied von mindestens 500 mbar kein Gasfluss entlang des Drahts durch die Schlauchwand auftritt. Vorzugsweise sind die Wandstärke des Körpers und seine Materialeigenschaften insbesondere hinsichtlich Materialwahl und Elastizität so beschaffen, dass auch größere Druckunterschiede von z.B. 1, 2, 3 oder 4 bar zu keinem Gasfluss entlang des Drahts durch die Schlauchwand führen.

Das Stichloch ist ein durch Materialverdrängung erzeugtes Loch. Vorzugsweise ist das Stichloch ausschließlich durch Materialverdrängung ohne Materialabtrag erzeugt. Es unterscheidet sich insoweit von einem Bohrloch oder Stanzloch, welches durch Abtrag oder Entfernung von Material gebildet wird. Das dagegen erfindungsgemäß in der flexiblen Wandung ausgebildete Stichloch ist von dem Draht elastisch aufgeweitet, so dass sich die flexible Wandung der Gasweiche an dem Stichloch abdichtend an den Draht anschmiegt. Erfindungsgemäß liegt die flexible Wandung an dem Stichloch elastisch gespannt an dem Draht an. Mit anderen Worten, die flexible Wandung steht an dem Stichloch unter einer elastischen Vorspannung. Das Stichloch hat die Tendenz sich zu schließen, wenn der Draht entfernt wird. Deswegen ist es möglich, den Draht dichtungslos durch das Stichloch zu führen. Weder innerhalb des Durchgangskanals noch außerhalb des elastischen Körpers ist an dem Draht ein Dichtungsmaterial nötig. Der elastische Körper dichtet an dem metallischen Draht selbst ab. Allerdings kann an dem Draht und dem elastischen Körper außen, wo der Draht aus dem elastischen Körper austritt, Dichtungsmaterial, beispielsweise in Gestalt eines Klebstoffs oder dergleichen, angebracht werden. Dies ist doch rein optional und in vielen Fällen überflüssig.

Der Draht ist vorzugsweise metallisch blank, d.h. ohne nichtmetallische Oberflächenbeschichtung. Er kann jedoch auch mit einer Beschichtung z.B. aus einem Kunststoff versehen sein, die fest an seiner Oberfläche haftet. Unabhängig davon ist es möglich, den Draht nach dem Durchstechen der Wandung zu erwärmen, um ihn mit der Wandung zu verschmelzen. Ist auf dem Draht eine nichtmetallische schmelzbare Beschichtung vorhanden, kann diese zum Beispiel durch energetische Einwirkung, durch Strahlung, Wärme, Ultraschall oder dergleichen, zumindest lokal aufgeschmolzen werden, um eine zusätzliche Abdichtung und/oder Verklebung mit dem Material der Wandung zu schaffen.

Vorzugsweise ist das Stichloch schräg zu der Wandung orientiert. So führt der Draht ebenfalls schräg zu der Wandung und somit auch schräg zu der Längsachse des elastischen Körpers durch die Wandung. Durch diese Maßnahmen, d.h. durch eine von der Radialrichtung abweichende Führung des Stichloches und des Drahts, wird die Berührungsfläche zwischen dem Draht und der Wandung des elastischen Körpers maximiert, was der Dichtungswirkung zugutekommt.

Der Winkel zwischen der Längsrichtung des Durchgangskanals und dem Stichloch bzw. dem durch das Stichloch geführten Draht ist jedoch vorzugsweise größer als 10°, größer als 20°, größer als 30° oder auch größer als 40°. Jedenfalls ist er aber kleiner oder gleich 90°, besser kleiner 80° oder, wie es bevorzugt wird, kleiner als 70°. Besonders bevorzugt ist, wenn der Winkel kleiner als 60° ist. Dies führt zu einer gasdichten Drahtdurchführung, die sich einfach herstellen lässt und dauerhaft verlässlich ist.

Vorzugsweise weist der Körper eine Flexibilität auf, die größer ist als die Flexibilität des Schlauchs. Damit schmiegt sich der flexible Körper an dem Schlauch abdichtend an. Der Schlauch ist dank seiner geringeren Flexibilität etwas knicksteif und lässt sich so in den Durchgangskanal des flexiblen Körpers einschieben. So kann der Innendurchmesser des Durchgangskanals (etwas) geringer sein, als der Außendurchmesser des Schlauchs. Der Schlauch ist nach dem Einschieben in den Durchgangskanal reibschlüssig in dem Körper gehalten. Zur Unterstützung der Dichtigkeit und des Reibschlusses kann in der Instrumentenanschlusseinrichtung eine Klemmvorrichtung vorgesehen sein, die den elastischen Körper lokal radial nach innen vorspannt und so die Pressung zwischen dem elastischen Körper und dem Schlauch erhöht.

Der elastische Körper kann aus einem Silikon-kunststoff bestehen. Der Schlauch kann aus einem anderen Kunststoff bestehen, beispielsweise Polyamid, Polyester, Polycarbonat, TPA, Pebax, Polyethylen, Polypropylen oder einem anderen geeigneten Kunststoff.

Ist der Draht in dem Schlauch axial fixiert, kann der Draht bei der Herstellung der Instrumentenanschlusseinrichtung mit dem Schlauch zusammen in den Körper eingeführt werden. Dies insbesondere, wenn der Draht knicksteif ist, beispielsweise indem er aus einem federnden knicksteifen Material, wie beispielsweise Stahldraht, besteht. Die Knicksteifigkeit ist bezüglich des Durchstechwiderstands gegeben, d.h. bezüglich der Längskraft, die der Draht erfährt, wenn er wie eine Nadel zum Durchstechen der Wandung des elastischen Körpers genutzt wird. Dabei ist er vorzugsweise wenigstens auf einer Länge von 1 cm bis 2 cm knicksteif. Vorzugsweise weist der Draht eine noch höhere Knicksteifigkeit auf, so dass er auch bei einer frei auskragenden Länge von mindestens 3, mindestens 4 oder mindestens 5 cm noch durch die Wand des Körpers sticht ohne auszuknicken. Bei der Herstellung der Gasdurchführung kann der Draht somit als Werkzeug zum Herstellen des Stichlochs benutzt werden. Vorzugsweise wird er dabei lediglich ein einziges Mal durch die Wandung gestochen und verbleibt dann in dem Stichloch ohne wieder entfernt zu werden.

Ist der Draht in dem Schlauch nicht axial fixiert, kann er bei der Herstellung der Instrumentenanschlusseinrichtung zunächst ohne den Schlauch in den Körper eingeführt werden. Nachdem er die Wandung des Körpers durchstochen hat, kann der Schlauch auf den Draht aufgefädelt und in den Körper eingeschoben werden. Wenn der Draht nackt, d.h. ohne Schlauch, durch die Wandung gestochen wird, ist es vorteilhaft, wenn er eine Knicksteifigkeit aufweist, die so hoch ist, dass eine außerhalb des Körpers auf den Draht aufgebrachte Schubkraft so auf die Drahtspitze aufgebracht wird, dass sie die Wandung durchsticht. Hat der Draht hingegen eine geringere Druck- bzw. Knicksteifigkeit, kann er mittels eines Werkzeugs in den Körper eingesetzt werden, der den Draht in einem Abstand zu seinem proximalen Ende fasst und nur einen zum Durchstechen der Wandung ausreichenden (kurzen) Abschnitt in proximaler Richtung frei ausragen lässt.

Das Verfahren zur Herstellung der Instrumentenanschlusseinrichtung sieht vor, den hohlzylindrischen Körper so zu positionieren, dass er an einer Stelle vorzugsweise um mindestens 30° gebogen ist, wobei sich von der Biegestelle ein gerader Abschnitt (Schenkel) des Körpers weg erstreckt. Der Schlauch wird dann mit dem aus ihm heraus ragenden Drahtende in den geraden Abschnitt des Körpers eingeschoben, wobei der Draht die Wandung des hohlzylindrischen Körpers an der Biegestelle durchsticht. Der Draht kann, falls gewünscht, an seinem stechenden Ende zugespitzt oder angeschärft, d.h. mit einer Nadelspitze oder einer Schneidkante versehen sein. Nach dem Durchstechen des hohlzylindrischen Körpers wird der Körper freigegeben, wodurch er in seine gestreckte Position zurückspringt. Somit ist die Gasweiche für die Instrumentenanschlusseinrichtung fertiggestellt. Das Verfahren kann insbesondere auch durchgeführt werden, wenn der Körper zum Durchstechen um mehr als 30°, z.B. 40°, 60°, 90°, 100°, 110°, 120° oder darüber abgewinkelt wird.

Nach dem Durchstechen der Wandung des flexiblen hohlzylindrischen Körpers kann der Draht etwas erwärmt werden, beispielsweise um an dem Stichloch eine Verklebung zwischen dem Material des hohlzylindrischen Körpers und der Oberfläche des Drahts herbeizuführen. Dies ist jedoch optional und abhängig von den Materialeigenschaften des hohlzylindrischen Körpers.

Weitere Einzelheiten vorteilhafter Details der Erfindung ergeben sich aus Ansprüchen sowie der Figurenbeschreibung, zu der die Zeichnung mit folgenden Figuren gehört:
Figur 1 ein Instrument mit einer Instrumentenanschlusseinrichtung, in perspektivischer schematisierter Darstellung,
Figur 2 die Instrumentenanschlusseinrichtung nach Figur 1, in geöffneter, teilweise geschnittener Darstellung,
Figur 3 die Gasweiche der Instrumentenanschlusseinrichtung nach Figur 2, in teilweiser geschnittener ausschnittsweiser Darstellung,
Figur 4 ein Ausschnitt aus der Wandung des hohlzylindrischen Körpers der Gasweiche mit einem Stichloch,
Figur 5 den Ausschnitt aus der Wandung nach Figur 4 mit einem Stichloch in den ein Draht steckt,
Figur 6 die Instrumentenanschlusseinrichtung bei der Erzeugung des Stichlochs, in teilweiser längsgeschnittener schematischer Darstellung,
Figur 7 die Instrumentenanschlusseinrichtung nach Figur 6, nach der Herstellung einer Drahtdurchführung für den Draht,
Figur 8 ein abgewandelte Ausführungsform der Instrumentenanschlusseinrichtung ähnlich Figur 2, jedoch mit Gasfilter.

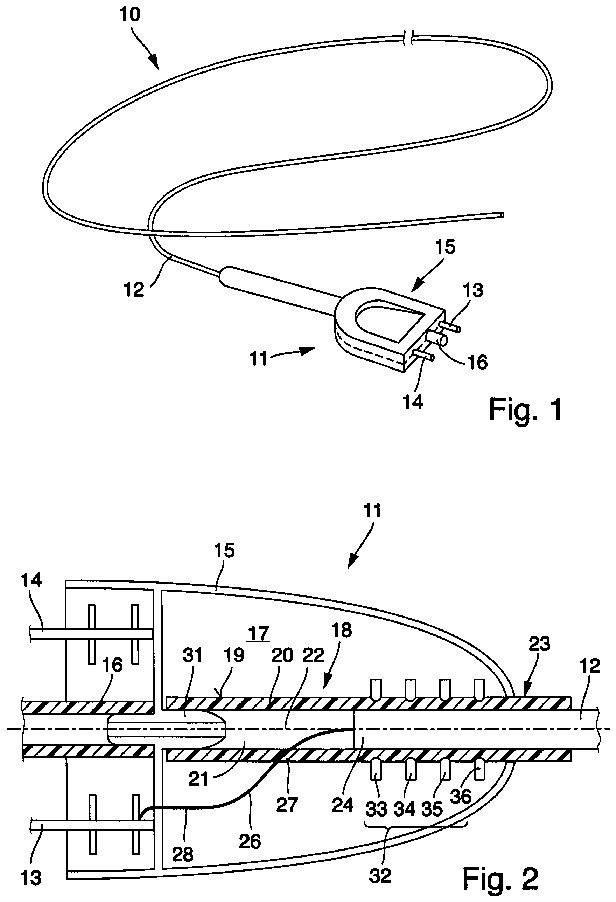

Figur 1 veranschaulicht ein Instrument 10 in Gestalt einer flexiblen Sonde, wie sie beispielsweise zur endoskopischen Behandlung von menschlichen oder tierischen Patienten geeignet ist. Dieses Instrument 10 veranschaulicht die Erfindung lediglich beispielhaft. Die Erfindung kann gleichermaßen bei Instrumenten in anderweitiger Ausbildung, beispielsweise bei Instrumenten für offenchirurgischen Einsatz oder bei laparoskopischen Instrumenten Anwendung finden. Allen solchen Instrumenten ist jedoch gemeinsam, dass sich von einer Instrumentenanschlusseinrichtung 11 in distaler Richtung ein Schlauch 12 weg erstreckt, der ein Zuleitungsschlauch für das eigentliche Instrument 10 oder, wie es in Figur 1 dargestellt ist, Teil des Instruments selbst ist. Bei dem Beispiel nach Figur 1 bildet der Schlauch 12 das proximale Ende des Instruments. Bei anderen Instrumenten ist der Schlauch 12 ein Zuleitungsschlauch, der nicht zwingend als Teil des Instruments angesehen werden muss.

Die Instrumentenanschlusseinrichtung 11 dient der Versorgung des Instruments 10 sowohl mit einem gasförmigen Medium, wie beispielsweise Argon, einem anderen Inertgas, einem Reaktivgas oder auch einer Flüssigkeit, sowie auch mit Spannung und/oder Strom. Das beispielhaft in Figur 1 veranschaulichte Instrument ist eine Argon-Plasmasonde, die zum Betrieb mit Argon und elektrischem Wechselstrom zu versorgen ist. Die Instrumentenanschlusseinrichtung 11 eignet sich aber auch für andere Instrumente, denen über eine elektrische Leitung elektrische Leistung und über ein Schlauchlumen ein Gas (oder eine Flüssigkeit) zuzuführen ist. Die Erfindung ist insbesondere für solche Instrumente geeignet, bei denen die elektrische Leitung innerhalb des Schlauches 12 liegt.

Die Instrumentenanschlusseinrichtung 11 weist mindestens einen zum Beispiel stiftförmigen oder anderweitig ausgebildeten elektrischen Kontakt 13 zur Strom- oder Spannungsversorgung und gegebenenfalls einen oder mehrere weitere elektrische Kontakte 14 auf. Die elektrischen Kontakte 13, 14 können in einem Steckergehäuse 15 parallel zueinander gehaltene Stiftkontakte sein.

Die Instrumentenanschlusseinrichtung 11 umfasst auch eine Gasanschlussbuchse 16, die beispielsweise durch ein flexibles schlauchähnliches Buchsenstück gebildet sein kann, das in der Nähe der Kontaktstifte 13, 14, beispielsweise zwischen diesen steht.

Figur 2 veranschaulicht den Aufbau der Instrumentenanschlusseinrichtung 11 mit geöffnetem Steckergehäuse 15. Die dargestellte Gehäuseschale des Steckergehäuses 15 enthält die elektrischen, hier stiftförmigen, Kontakte 13, 14 sowie das Gasanschlussstück 16, die in oder an dem Steckergehäuse 15 ortsfest gehalten sind. Das Gasanschlussstück 16 kann als flexible Muffe ausgebildet sein. Das Steckergehäuse 15 umschließt außerdem einen Innenraum 17, in dem eine Gasweiche 18 angeordnet ist. Diese Gasweiche 18 dient dazu, die Stromversorgung und die Gasversorgung des Instruments 10 zusammenzuführen. Dazu weist die Gasweiche 18 einen hohlzylindrischen schlauchartigen Körper 19 mit einer flexiblen Wandung 20 auf. Der Körper 19 umschließt einen Durchgangskanal 21, dessen Längsmittelachse 22 vorzugsweise gerade (gestreckt) verläuft.

Die Wandung 20 besteht aus einem flexiblen, elastisch federnden Kunststoff, vorzugsweise einem Silikon-kunststoff. Das proximale Ende der Gasweiche 18 steht mit dem Gasanschlussstück 16 in Fluidverbindung. Es kann dazu eine entsprechende Gehäusestruktur (z.B. ein nach Art von Aufstecknippel ausgebildeter Fluidverbinder 31) vorgesehen sein.

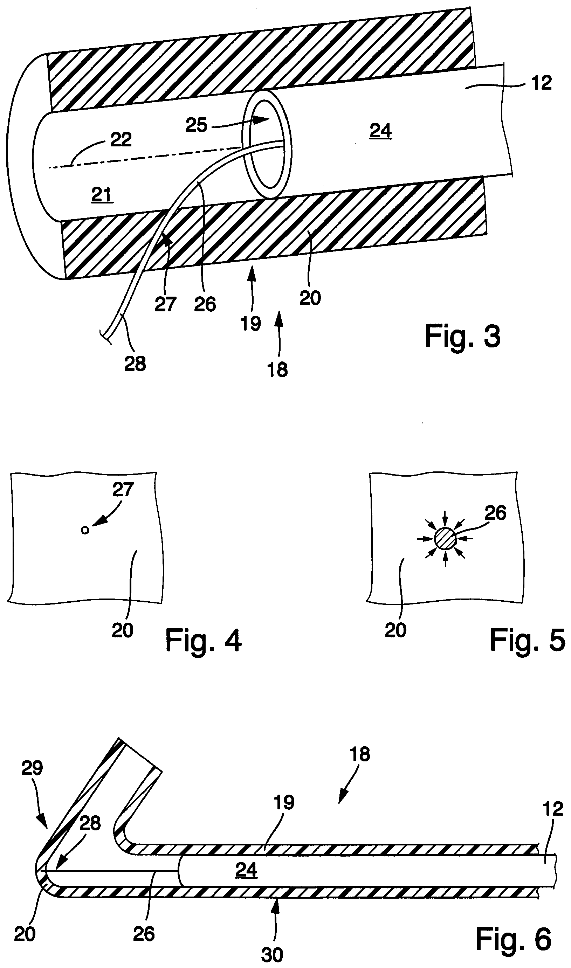

In dem distalen Ende 23 des Körpers 20, steckt das proximale Ende 24 des Schlauchs 12. Dieser umschließt, wie insbesondere aus Figur 3 ersichtlich, wenigstens ein Lumen 25, das sich vorzugsweise von dem proximalen Ende 24 des Schlauchs 12 bis zu dem distalen Ende des Schlauchs 12 oder auch des Instruments 10 erstreckt. Der Schlauch 12 kann auch mehrere sich parallel zueinander über die Länge des Schlauchs erstreckende Lumen aufweisen.

Das proximale Ende 24 des Schlauchs 12 steckt spielfrei und somit straff und spaltlos an der Wandung 20 des flexiblen Körpers 19 anliegend in dem Durchgangskanal 21. Damit ist eine gasdichte Verbindung zwischen dem Durchgangskanal 21 und dem Lumen 25 geschaffen.

In dem Lumen 25 ist ein Draht 26 angeordnet, der aus dem proximalen Ende 24 des Schlauchs 12 herausragt und die Wandung 20 bei einem Stichloch 27 durchquert. Alternativ kann der Draht in das Kunststoffmaterial des Schlauchs 12 eingebettet sein. Ist der Schlauch 12 mehrlumig, kann der Draht 26 ebenfalls durch eines der Lumen oder durch das Material des Schlauchs 12 verlaufend angeordnet sein.

Der Draht 26 ist vorzugsweise ein knicksteifer Draht, wie beispielsweise ein Federstahldraht oder ein Draht aus einem anderen federnden, knicksteifen Material. Die Knicksteifigkeit des Drahts 26 ist vorzugsweise so hoch, dass der Draht 26 mindestens bei einer frei auskragenden Länge von 1 cm bis 2 cm in der Lage ist, die Wandung 20 zu durchstechen, wenn er auf diese hin geschoben wird. Dies vorzugsweise auch dann, wenn das stirnseitige Ende des Drahts 26 nicht gesondert geschärft worden ist, sondern lediglich eine Scher- oder Bruchfläche aufweist. Es versteht sich somit, dass der Begriff der Knicksteifigkeit von der Durchstechfestigkeit der Wandung 20 und somit von der Materialbeschaffenheit der Wandung 20, deren Dicke sowie der gewählten freien Auskraglänge des Draht 26 abhängt. Federstahldraht mit einem Durchmesser von 0,1 mm bis 0,2 mm ist aber ausreichend knicksteif bei üblichen flexiblen Silikonmaterialien und Wandstärken bis zu mehreren Millimetern bei Auskraglängen von bis zu 2 cm oder mehr. Besonders leicht lässt sich das erfindungsgemäße Verfahren mit einem Draht ausführen, der so knick- und biegesteif ist, dass er auch eine freie Auskraglänge von mindestens 3, mindestens 4 oder mindestens 5 cm zulässt.

Der Draht 26 kann über seine gesamte Länge sich durch das Lumen 25 erstreckend einheitlich ausgebildet sein oder außerhalb oder innerhalb der Gasweiche 18 Verbindungsstellen aufweisen und somit abschnittsweise auch aus anderen Materialien bestehen. Außerdem kann der Draht 26 oberflächenbeschichtet sein, beispielsweise ganz oder teilweise eine Silberbeschichtung oder eine andere Metallbeschichtung, beispielsweise eine Kupferbeschichtung aufweisen. Auch ist es möglich, den Draht (z.B. nackter Stahldraht oder metallbeschichteter Stahldraht) zusätzlich mit einer nichtmetallischen, fest an seiner Oberfläche haftenden Beschichtung, insbesondere einer thermoplastischen Beschichtung zu versehen. Die nichtmetallische Beschichtung kann sich über die gesamte Länge des Drahts oder alternativ auch nur über einen Abschnitt seiner Länge, z.B. über den aus dem Schlauch 12 heraus ragenden Teil erstrecken.

Das Stichloch 27 ist vorzugsweise von dem Draht 26 selbst gestochen und somit ohne Materialabtrag erzeugt. Würde der Draht 26 aus dem Stichloch 27 entfernt, würde es sich unter elastischer Entspannung der Wandung 20, wie es Figur 4 zeigt, zumindest nahezu oder auch ganz wieder schließen. Wenn sich der Draht 26 aber, wie es Figur 5 zeigt, in dem Stichloch 27 befindet, liegt die Wandung 20 unter Vorspannung an dem Draht 26 an und dichtet somit an diesem ab. So bildet die Wandung des Stichlochs 27 mit dem Draht 26 eine gasdichte Drahtdurchführung.

Vorzugsweise ist das Stichloch 27 in einem spitzen Winkel zu der Längsachse 22 des Durchgangskanals 21 angeordnet. Der zwischen dem Stichloch 27 und der Längsmittelachse 22 eingeschlossenen Winkel ist dabei kleiner oder gleich 90°, vorzugsweise kleiner als 80° weiter vorzugsweise kleiner als 70° und am besten kleiner als 60°. Andererseits ist der Winkel größer als 10°, vorzugsweise größer als 20° besser größer als 30° und vorzugsweise größer als 40°. Mit dieser Bemessung werden eine einfache Herstellbarkeit und zugleich eine gute Dichtigkeit der Drahtdurchführung erreicht.

Der Draht 26 kann in dem Lumen 25 frei liegen, so dass keine axiale feste Verbindung zwischen dem Draht 26 und dem Schlauch 12 besteht. Der Draht 26 kann aber auch axial fest mit dem Schlauch 12 verbunden sein, beispielsweise durch entsprechende in dem Lumen 25 angeordnete Halter oder Strukturen des Schlauchs 12.

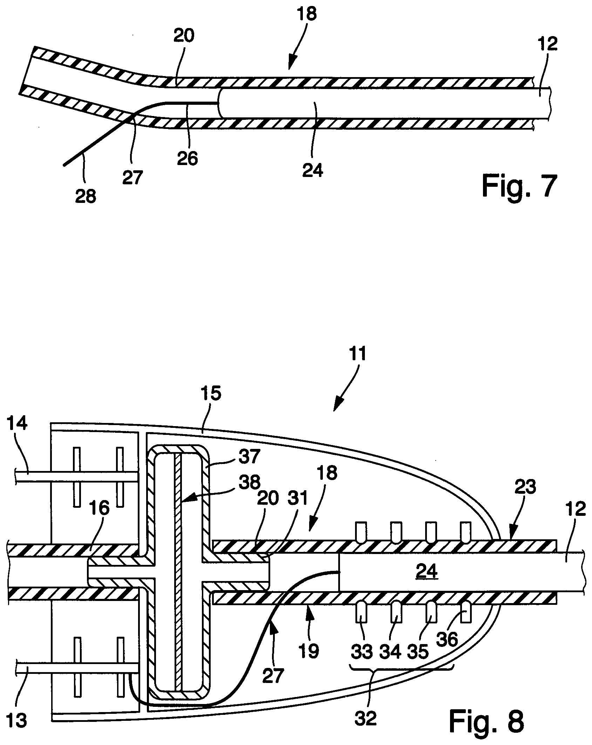

Zur weiteren Veranschaulichung der Erfindung veranschaulichen die Figuren 6 und 7 wesentliche Herstellungsschritte der Gasweiche 18:

Zur Herstellung der Gasweiche 18 werden zunächst der hohlzylindrische Körper 19 und der Draht 26 bereitgestellt. Der Draht 26 kann nackt oder, falls er mit dem Schlauch 12 verbunden ist, auch zusammen mit dem Schlauch 12 bereitgestellt werden, wobei jedoch das proximale Ende 28 des Drahts 26 um einen gewünschten Betrag von beispielsweise 1 cm bis 2 cm oder auch mehreren Zentimetern aus dem proximalen Ende 24 des Schlauchs 12 herausragt.

Der Körper 19 wird nun in die in Figur 6 veranschaulichte abgewinkelte Form gebracht, bei der eine Biegestelle 29 gebildet ist, an der der schlauchartige Körper 19 um 30° oder mehr abgewinkelt ist. Bevorzugt ist eine Abwinklung von mehr als 90°. Von der Biegestelle 29 erstreckt sich mindestens ein gerader Schenkel 30 weg, der zur Aufnahme des proximalen Endes 24 des Schlauchs 12 vorgesehen ist.

Der Draht 26 wird nun so in den Schenkel 30 eingeführt, dass sein proximales Ende 28 vorzugsweise unter einem etwa rechten Winkel auf die dortige Stelle der Wandung 20 trifft. Weiterer Vorschub des Drahts 26 bewirkt, dass der Draht 26 die Wandung 20 unter Ausbildung des Stichlochs 27 durchsticht. Zugleich oder danach wird der Schlauch 12 mit seinem proximalen Ende 24 in den Schenkel 30 eingeführt.

Nach Durchführung dieser Operation wird die insoweit fertiggestellte Gasweiche 18 einer Haltevorrichtung entnommen, so dass sich die Biegestelle 29 entspannen und wieder strecken kann. Die Gasweiche 18 nimmt dann etwa die in Figur 7 veranschaulichte Form an. Je nach Federkonstante des Drahts 26 und der Wandung 20 ist diese wieder vollkommen hohlzylindrisch gerade oder wie es Figur 7 veranschaulicht noch leicht abgewinkelt. Jedenfalls aber steckt der Draht 26 fluiddicht in dem Stichloch 27.

Im nächsten Schritt kann nun die Gasweiche 18 in den Steckergehäuse 15 montiert werden. Dazu wird der Körper 19 mit seinem proximalen Ende, wie es Figur 2 nahelegt, auf den Fluidverbinder 31 aufgeschoben, der als Gehäusestruktur ausgebildet sein kann und die Fluidverbindung zu dem Gasanschlussstück 16 herstellt. Außerdem kann das proximale Ende des Drahts 28 mit dem Kontakt 13 oder 14 (oder mit beiden) elektrisch und mechanisch verbunden beispielsweise verlötet, verschweißt, vercrimpt oder anderweitig verbunden werden. Außerdem wird die Gasweiche 18 zumindest dort, wo der Körper 19 das proximale Ende 24 des Schlauchs 12 aufgenommen hat, in eine Klemmstruktur 32 eingelegt. Diese kann aus einer oder mehreren Wandabschnitten 33, 34, 35, 36 bestehen, die fester Bestandteil der Gehäuseschale des Steckergehäuses 15 sein können und jeweils einen U-förmigen Ausschnitt aufweisen, dessen lichte Weite etwas geringer ist als der Außendurchmesser des Körpers 19. Dieser wird durch die Wandabschnitte 33 bis 36 so radial nach innen verformt, so dass das proximale Ende 24 des Schlauchs 12 innerhalb der Klemmvorrichtung 32 in dem Körper 19 festgeklemmt ist. Zugleich ist der Körper 19 zugfest in dem Gehäuse 15 gesichert. Vorzugsweise ist die Klemmstruktur in axialer Richtung elastisch nachgiebig. Die Stegbreite der Wandabschnitte 33, 34, 35, 36 ist geringer, als 1/5-tel, 1/7-tel oder 1/10-tel des Außendurchmessers des flexiblen Körpers. Hierdurch entsteht eine verbesserte Fixierung der Gasweiche in der Halbschale.

In Figur 2 ist lediglich eine untere Gehäuseschale veranschaulicht. Die abgenommene obere Gehäuseschale kann als Klemmstruktur eben solche Wandabschnitte aufweisen, die zwischen die aus Figur 2 ersichtlichen Wandabschnitte 33 bis 36 fassen und somit die Klemmung des Schlauchs 12 in der Gasweiche 18 ergänzen.

Figur 8 veranschaulicht eine erweiterte Ausführungsform der Erfindung, bei der der Fluidverbinder 31 Bestandteil eines Filtergehäuses 37 mit einem darin vorgesehenen Gasfilter 38 ist. Der Gasfilter 38 kann ein feinporiger Körper sein, der sowohl den Übertritt von Verschmutzungen vom speisenden Gerät in das Instrument 10 als auch die Rückübertragung von Verschmutzungen von dem Instrument 10 auf das speisende Gerät verhindert. Im Übrigen gilt die vorige im Zusammenhang mit den Figuren 1 bis 7 gegeben Beschreibung unter Zugrundelegung gleicher Bezugszeichen entsprechend.

Eine erfindungsgemäße Instrumentenanschlusseinrichtung 11 weist einen flexiblen schlauchartigen Körper 19 mit flexibler Wandung 20 auf, in dem das proximale Ende 24 eines zu dem Instrument 10 gehörigen oder zu diesem führenden Schlauchs 12 steckt. Der Schlauch 12 weist ein Lumen 25 auf, das längs durch den Schlauch 12 führt. Außerdem ist in dem Schlauch 12 zum Beispiel in dem Lumen 25 ein Draht 26 angeordnet. Dieser führt durch ein Stichloch 27, das der Draht 26 beim Durchstechen der Wandung 20 selbst erzeugt hat. Das Stichloch 27 ist vorzugsweise gerade und führt schräg, d.h. sowohl zur radialen als auch zur Längsmittelachse 22 geneigt durch die Wandung 20.

### Bezugszeichen:

- 10: Instrument
- 11: Instrumentenanschlusseinrichtung
- 12: Schlauch
- 13, 14: elektrischer Kontakt
- 15: Steckergehäuse
- 16: Gasanschlussstück
- 17: Innenraum
- 18: Gasweiche
- 19: Körper
- 20: Wandung
- 21: Durchgangskanal
- 22: Längs-Mittelachse
- 23: distales Ende des Körpers 19
- 24: proximales Ende des Schlauchs 12
- 25: Lumen des Schlauchs 12
- 26: Draht
- 27: Stichloch
- 28: proximales Ende des Drahtes 26
- 29: Biegestelle
- 30: Schenkel
- 31: Fluidverbinder
- 32: Klemmstruktur
- 33- 36: Wandabschnitte
- 37: Filtergehäuse
- 38: Gasfilter

## Patentansprüche

1. Instrumentenanschlusseinrichtung (11), insbesondere für Instrumente (10), die einer Gaszufuhr und einer Stromzufuhr bedürfen,
mit einer Gasweiche (18), die einen hohlzylindrischen Körper (19) mit einer flexiblen Wandung (20) aufweist, die einen Durchgangskanal (21) begrenzt und die ein Loch aufweist,
mit einem Schlauch (12), der wenigstens ein Lumen (25) aufweist und sich mit einem Ende (24) in proximaler Richtung in den Körper (19) erstreckend in diesem an der Wandung (20) anliegend angeordnet ist,
mit einem Draht (26), der in dem Schlauch (12) in proximaler Richtung aus diesem herausführend angeordnet ist und sich durch das Loch erstreckt, **dadurch gekennzeichnet, dass** das Loch ein Stichloch (27) ist, das von dem Draht (26) elastisch aufgeweitet ist und dass die flexible Wandung (20) an dem Stichloch (27) elastisch gespannt an dem Draht (26) anliegt.

2. Instrumentenanschlusseinrichtung nach Anspruch 1, **dadurch gekennzeichnet, dass** der Draht (26) dichtungslos durch das Stichloch (27) führt.

3. Instrumentenanschlusseinrichtung nach einem der vorstehenden Ansprüche, **dadurch gekennzeichnet, dass** das Stichloch (27) rechtwinklig oder schräg zu der Wandung (20) orientiert ist.

4. Instrumentenanschlusseinrichtung nach einem der vorstehenden Ansprüche, **dadurch gekennzeichnet, dass** das Stichloch (27) in einem Winkel zu einer von dem Durchgangskanal (21) bestimmten Längsrichtung (22) angeordnet ist, der kleiner als 90°, 80°, 70° oder kleiner als 60° ist, und/oder dass das Stichloch (27) in einem Winkel zu der Längsrichtung (22) angeordnet ist, der größer als 10°, 20°, 30° oder größer als 40° ist.

5. Instrumentenanschlusseinrichtung nach einem der vorstehenden Ansprüche, **dadurch gekennzeichnet, dass** der Körper (19) eine Flexibilität aufweist, die größer ist als die Flexibilität des Schlauchs (12).

6. Instrumentenanschlusseinrichtung nach einem der vorstehenden Ansprüche, **dadurch gekennzeichnet, dass** der Schlauch (12) in dem Körper (19) reibschlüssig gehalten ist.

7. Instrumentenanschlusseinrichtung nach einem der vorstehenden Ansprüche, **dadurch gekennzeichnet, dass** der Körper (19) aus einem Silikon-Kunststoff besteht.

8. Instrumentenanschlusseinrichtung nach einem der vorstehenden Ansprüche, **dadurch gekennzeichnet, dass** der Schlauch (12) aus einem Polyamid, einem Polyester, einem Polycarbonat, TPA, Pebax, Polypropylen oder einem Polyethylen besteht.

9. Instrumentenanschlusseinrichtung nach einem der vorstehenden Ansprüche, **dadurch gekennzeichnet, dass** der Draht (26) in dem Schlauch (12) fixiert ist.

10. Instrumentenanschlusseinrichtung nach einem der vorstehenden Ansprüche, **dadurch gekennzeichnet, dass** der Draht (26) ein federnder Draht, insbesondere ein Stahldraht ist.

11. Instrumentenanschlusseinrichtung nach einem der vorstehenden Ansprüche, **dadurch gekennzeichnet, dass** der Draht (26) aus einem federnden knicksteifen Material besteht.

12. Instrument (10) mit einer Instrumentenanschlusseinrichtung (11) nach einem der vorstehenden Ansprüche.

13. Verfahren zur Herstellung einer Instrumentenanschlusseinrichtung (11) nach einem der vorstehenden Ansprüche, **dadurch gekennzeichnet, dass** zunächst der Schlauch (12) mit dem aus seinem proximalen Ende (24) herausragenden Draht (26) sowie der hohlzylindrische Körper (19) bereitgestellt werden,
wonach der hohlzylindrische Körper (19) an einer Stelle (29) elastisch gebogen wird, wobei sich von der Stelle (29) mindestens ein gerader Abschnitt (30) des Körpers (19) weg erstreckt,
wonach das proximale Ende (28) des Drahts (26) in den geraden Abschnitt (30) eingeschoben wird, wobei der Draht (26) den Körper (19) an der gebogenen Stelle (29) durchsticht,
wonach der Körper (19) in seine entspannte, gestreckte Form rücküberführt wird.

## Claims

1. An instrument connection device (11), in particular for instruments (10) that require a gas supply and a power supply,
having a gas junction (18), which comprises a hollow cylindrical body (19) with a flexible wall (20) that delimits a through channel (21) and that comprises a hole,
having a hose (12), which comprises at least one lumen (25) and is arranged with one end (24) extending in proximal direction into the body (19), in abutment against the wall (20),
having a wire (26), which is arranged in the hose (12) projecting in proximal direction therefrom and which extends through the hole, **characterized in that** the hole is a punctured hole (27) which is elastically widened by the wire (26) and that the flexible wall (20) abuts against the wire (26) at the punctured hole (27) in an elastically tensioned manner.

2. The instrument connection device according to claim 1, **characterized in that** the wire (26) is guided through the punctured hole (27) without a seal.

3. The instrument connection device according to anyone of the preceding claims, **characterized in that** the punctured hole (27) is orientated at a right angle or obliquely to the wall (20).

4. The instrument connection device according to anyone of the preceding claims, **characterized in that** the punctured hole (27) is arranged at an angle relative to a longitudinal direction (22) defined by the through channel (21) which is less than 90°, 80°, 70° or less than 60°, and/or that the punctured hole (27) is arranged at an angle relative to the longitudinal direction (22) which is greater than 10°, 20°, 30° or greater than 40°.

5. The instrument connection device according to anyone of the preceding claims, **characterized in that** the body (19) has a flexibility that is greater than the flexibility of the hose (12).

6. The instrument connection device according to anyone of the preceding claims, **characterized in that** the hose (12) is held inside the body (19) in a friction-fit manner.

7. The instrument connection device according to anyone of the preceding claims, **characterized in that** the body (19) is made of a silicone plastic.

8. The instrument connection device according to anyone of the preceding claims, **characterized in that** the hose (12) consists of a polyamide, polyester, polycarbonate, TPA, Pebax, polypropylene or polyethylene.

9. The instrument connection device according to anyone of the preceding claims, **characterized in that** the wire (26) is fixed inside the hose (12).

10. The instrument connection device according to anyone of the preceding claims, **characterized in that** the wire (26) is a springy wire, in particular a steel wire.

11. The instrument connection device according to anyone of the preceding claims, **characterized in that** the wire (26) consists of a springy kink-resistant material.

12. An instrument (10) having an instrument connection device (11) according to anyone of the preceding claims.

13. A method for manufacturing an instrument connection device (11) according to anyone of the preceding claims, **characterized in that** the hose (12) with the wire (26) projecting from its proximal end (24) and the hollow cylindrical body (19) are first provided,
whereafter the hollow cylindrical body (19) is elastically bent at a site (29), whereby at least one straight section (30) of the body (19) extends away from the site (29),
whereafter the proximal end (28) of the wire (26) is inserted into the straight section (30), whereby the wire (26) pierces the body (19) at the bent site (29),
whereafter the body (19) is transferred back into its relaxed, stretched shape.

## Revendications

1. Dispositif de raccordement d'instruments (11), en particulier pour les instruments (10) qui nécessitent une alimentation en gaz et une alimentation électrique,
avec un déflecteur de gaz (18) qui présente un corps cylindrique creux (19) avec une paroi flexible (20) qui délimite un canal de passage (21) et qui présente un trou, avec un tuyau (12) qui présente au moins une lumière (25) et est disposé de manière à s'étendre avec une extrémité (24) dans la direction proximale dans le corps (19) et à être en appui contre la paroi (20) dans celui-ci,
avec un fil (26) qui est disposé dans le tuyau (12) de manière à sortir de celui-ci dans la direction proximale et s'étend à travers le trou,
**caractérisé en ce que** le trou est un trou de piqûre (27), qui est élastiquement élargi par le fil (26) et que
la paroi flexible (20) est en appui sur le trou de piqûre (27) sous tension élastique sur le fil (26).

2. Dispositif de raccordement d'instruments selon la revendication 1,
**caractérisé en ce que** le fil (26) passe sans joint à travers le trou de piqûre (27).

3. Dispositif de raccordement d'instruments selon l'une quelconque des revendications précédentes, **caractérisé en ce que** le trou de piqûre (27) est orienté perpendiculairement ou obliquement par rapport à la paroi (20).

4. Dispositif de raccordement d'instruments selon l'une quelconque des revendications précédentes, **caractérisé en ce que** le
trou de piqûre (27) est disposé à un angle par rapport à une direction
longitudinale (22) définie par le canal de passage (21) qui est inférieur à 90°, 80°, 70° ou inférieur à 60°, et/ou que le trou de piqûre (27) est disposé à un angle par rapport à la direction longitudinale (22) qui est supérieur à 10°, 20°, 30° ou supérieur à 40°.

5. Dispositif de raccordement d'instruments selon l'une quelconque des revendications précédentes, **caractérisé en ce que** le corps (19) présente une flexibilité qui est supérieure à la flexibilité du tuyau (12).

6. Dispositif de raccordement d'instruments selon l'une quelconque des revendications précédentes, **caractérisé en ce que** le tuyau (12) est maintenu par friction dans le corps (19).

7. Dispositif de raccordement d'instruments selon l'une quelconque des revendications précédentes, **caractérisé en ce que** le corps (19) est constitué d'un plastique siliconé.

8. Dispositif de raccordement d'instruments selon l'une quelconque des revendications précédentes, **caractérisé en ce que** le tuyau (12) est constitué d'un polyamide, d'un polyester, d'un polycarbonate, de TPA, Pebax, polypropylène ou d'un polyéthylène.

9. Dispositif de raccordement d'instruments selon l'une quelconque des revendications précédentes, **caractérisé en ce que** le fil (26) est fixé dans le tuyau (12).

10. Dispositif de raccordement d'instruments selon l'une quelconque des revendications précédentes, **caractérisé en ce que** le fil (26) est un fil à ressort, en particulier un fil d'acier.

11. Dispositif de raccordement d'instruments selon l'une quelconque des revendications précédentes, **caractérisé en ce que** le fil (26) est constitué d'un matériau élastique résistant à la flexion.

12. Instrument (10) avec un dispositif de raccordement d'instrument (11) selon l'une quelconque des revendications précédentes.

13. Procédé pour la fabrication d'un dispositif de raccordement d'instruments (11) selon l'une quelconque des revendications précédentes, **caractérisé en ce que** le tuyau (12) est d'abord fourni avec le fil (26) faisant saillie de son extrémité proximale (24) ainsi que le corps cylindrique creux (19),
après quoi le corps cylindrique creux (19) est plié élastiquement à un endroit (29), dans lequel au moins une section droite (30) du corps (19) s'étend à l'écart de l'endroit (29),
après quoi l'extrémité proximale (28) du fil (26) est insérée dans la section droite (30), dans lequel le fil (26) perce le corps (19) à l'endroit (29) courbé,
après quoi le corps (19) est ramené dans sa forme détendue, étirée.
